# EUROPEAN PATENT SPECIFICATION

(11) **EP 4 526 682 B1**
(45) Date of publication and mention of the grant of the patent: **26.08.2026**
(21) Application number: 23726544.2
(22) Date of filing: 19.05.2023
(51) Int. Cl.: G01N 33/575

(54) **METHOD FOR IDENTIFYING CANCER PATIENTS THAT BENEFIT FROM ANTI-CLEVER-1 TREATMENT**
VERFAHREN ZUR IDENTIFIZIERUNG VON KREBSPATIENTEN, DIE VON EINER ANTI-CLEVER-1-BEHANDLUNG PROFITIEREN
PROCÉDÉ D'IDENTIFICATION DE PATIENTS ATTEINTS D'UN CANCER QUI BÉNÉFICIENT D'UN TRAITEMENT ANTI-CLEVER-1

(30) Priority: 20.05.2022 FI 20225445
(43) Date of publication of application: 26.03.2025
(73) Proprietor: Faron Pharmaceuticals OY, 20520 Turku (FI)
(72) Inventor: JALKANEN, Juho, 23120 Mietoinen (FI); BJÖRKMAN, Mari, 20720 Turku (FI); HOLLMÉN, Maija-Leena, 20760 Piispanristi (FI)
(74) Representative: Berggren Oy
(86) International application number: PCT/FI2023/050279
(87) International publication number: WO 2023/222952

(56) References cited:
- WO-A1-2010/122217
- WO-A1-2021/058866
- WO-A1-2021/094652
- ANONYMOUS: "Product Datasheet Stabilin-1 Antibody (4G9) H00023166-M05", 22 August 2021 (2021-08-22), pages 1 - 4, XP093059143, Retrieved from the Internet <URL:https://www.novusbio.com/PDFs/H00023166-M05.pdf> [retrieved on 20230630]
- BJÖRKMAN MARI L ET AL: "Abstract 4334: Clever-1/PD-L1 ratio predicts response to Bexmarilimab, a novel macrophage-guided immunotherapy, in immune deprived cancers", AACR ANNUAL MEETING 2023, vol. 83 (7 Supp.), 1 April 2023 (2023-04-01), pages 1 - 1, XP093059892, DOI: 10.1158/1538-7445.AM2023-4334

## Description

### Field of the invention

The present invention relates to the pre-treatment identification of cancer patients that respond to a treatment comprising an administration of an agent capable of binding to Common Lymphatic Endothelial and Vascular Endothelial Receptor-1 (CLEVER-1) using immunohistochemistry staining of a tumor biopsy prior to the treatment.

### Background of the invention

Currently, immune checkpoint inhibitors targeting CTLA-4 and the PD-1/PD-L1 axis are approved for clinical use, and while highly efficacious in about 10-20 % of patients, the majority of cancer patients do not respond to checkpoint inhibitors and other novel immunotherapies. A key to success in immunotherapy is to identify the patients that will respond [1]. Patients responding favorably to checkpoint inhibition usually have a pre-existing antitumor immune response, which is characterized by high density of IFNγ-producing CD8⁺ T cells, expression of PD-L1 in tumor-infiltrating immune cells, and high mutational load. PD-L1 expression in a pre-treatment biopsy is often used to pick patients for anti-PD-1 or anti-PD-L1 treatment.

Anti-CLEVER-1 is a novel immunotherapy targeting tumor associated macrophages, which has shown promising single agent efficacy in a first-in-human (FiH) clinical trial named MATINS (clinicaltrials.gov NCT03733990: A Study to Evaluate Safety, Tolerability and Preliminary Efficacy of FP-1305 in Cancer Patients (MATINS)) [2].

Innate immune cells such as macrophages, can dampen T cell activation and contribute to tumor progression despite high mutational load. The macrophages that contribute to tumor-related immunosuppression and provide tumor growth supporting signals may be highly eligible candidates for targeted therapies, since these cells are abundantly present in various tumors, they are very plastic and can be converted into pro-inflammatory macrophages supporting T cell activation and tumor killing [3, 4]. CLEVER-1 (also known as STABILIN-1) is a multifunctional molecule conferring scavenging ability on a subset of anti-inflammatory macrophages [5, 6]. However, there is still a need to identify the patients that will respond to anti-CLEVER-1 therapy and to find methods for the pre-treatment identification and patient selection for reducing or even eliminating un-necessary treatments.

WO 2010/122217 A1 discloses a method for screening of cancer patients that would respond to anti-CLEVER-1 therapy, based on the detection of the level of CLEVER-1 protein in a tumor sample obtained from a cancer patient by immunohistochemical methods.

### Summary of the Invention

It has been found out that cancer patients with low PD-L1 expression in a tumor biopsy respond to CLEVER-1 inhibition, which is totally opposite to prior art concerning checkpoint inhibitors. Further, it has now been found that the best way to predict response to a treatment based on CLEVER-1 inhibition is to use a combination of PD-L1 and CLEVER-1 expressions and/or a ratio of PD-L1 and CLEVER-1 expression in immunohistochemistry (IHC) staining of a tumor biopsy prior to the beginning of anti-CLEVER-1 therapy. Clinical benefit from anti-CLEVER-1 treatment has been seen in patients which PD-L1 expression is low and intra-tumoral CLEVER-1 expression is high in a pre-treatment tumor biopsy. The present invention discloses the detailed method for the evaluation of the ratio of PD-L1 expression and CLEVER-1 expression and provides a tool for patient selection.

Therefore, an object of the present invention to provide a novel method for the pre-treatment identification and patient selection of cancer patients that respond to an anti-CLEVER-1 therapy using immunohistochemistry staining of a tumor biopsy prior to the beginning of the anti-CLEVER-1 therapy, and thus reduce or even eliminate un-necessary treatments. More particularly, the present invention provides a method for the pre-treatment identification and patient selection of cancer patients that respond to an anti-CLEVER-1 treatment comprising an administration of anti-CLEVER-1 antibody, preferably anti-CLEVER-1 antibody bexmarilimab. By the method according to the invention, cancer patients to be treated by anti-CLEVER-1 therapy can be chosen and the low ratio of PD-L1 expression and CLEVER-1 intra-tumoral expression in immunohistochemistry staining of a tumor biopsy prior to the beginning of anti-CLEVER-1 therapy is an indication that the cancer patient is responsive to the anti-CLEVER-1 treatment.

In order to achieve among others, the objects presented above, the invention is characterized by what is presented in the characterizing parts of the enclosed independent claims. Some preferred embodiments of the invention will be described in the other claims. The embodiments and advantages mentioned in this disclosure are applicable, both to the method and to the uses according to the invention, even though it is not always specifically mentioned.

A typical method according to the present invention for pre-treatment identification of cancer patients that respond to anti-CLEVER-1 treatment comprising an administration of an agent capable of binding to CLEVER-1 in a patient, preferably an administration of anti-CLEVER-1 antibody, the method comprises
- providing a tumor sample obtained from a cancer patient,
- detecting the presence of PD-L1 expressing cells and CLEVER-1 expressing cells in the tumor sample by immunohistochemistry staining by a PD-L1 specific antibody and a mouse monoclonal IgG2a kappa STAB-1 antibody (clone 4G9), and
- calculating a percentage of PD-L1 expressing cells from the total amount of viable cells present in the stained sample, and calculating a percentage of intra-tumoral CLEVER-1 expressing cells from the total amount of viable intra-tumoral cells present in the stained sample,
wherein the tumor sample which shows low percentage of PD-L1 expressing cells or not comprising PD-L1 expressing cells together with substantial percentage of CLEVER-1 expressing intra-tumoral cells is an indication that the cancer patient is responsive to the anti-CLEVER-1 treatment. More particularly, the stained tumor sample, which shows 0 - 2 % of PD-L1 expressing cells, calculated from the total amount of viable cells present in the stained sample, together with at least 1 % of CLEVER-1 expressing intra-tumoral cells, calculated from the total amount of viable intra-tumoral cells present in the stained sample, is an indication that the cancer patient is responsive to the anti-CLEVER-1 therapy, typically anti-CLEVER-1 antibody therapy.

A method for treating a cancer patient is disclosed, which method comprises an administration of a therapeutically effective amount of anti-CLEVER-1 antibody, preferably anti-CLEVER-1 antibody bexmarilimab for use in a treatment of cancer in a patient having diagnosed with a tumour which shows no expression or low expression of PD-L1 (percentage of positive cells from all viable cells) together with substantial intra-tumoral expression (percentage of positive cells from all viable intratumoral cells) of CLEVER-1. More particularly, the present invention relates to anti-CLEVER-1 antibody, preferably anti-CLEVER-1 antibody bexmarilimab for use in a treatment of cancer in a patient having diagnosed with a tumour which shows low PD-L1 expressing cells and intra-tumoral CLEVER-1 expressing cells ratio in a sample obtained from the tumor. The anti-CLEVER-1 antibody, preferably anti-CLEVER-1 antibody bexmarilimab is used in treating cancer by reducing malignant tumour growth in a patient and/or by inhibiting metastasis formation.

A method for treating cancer patient is disclosed, which method comprises at least the following steps of
- obtaining a tumor sample from a cancer patient,
- detecting the presence of PD-L1 expressing cells and CLEVER-1 expressing cells in the tumor sample by immunohistochemistry staining by a PD-L1 specific antibody and a mouse monoclonal IgG2a kappa STAB-1 antibody (clone 4G9),
- calculating a percentage of PD-L1 expressing cells from the total amount of viable cells present in the stained sample, and calculating a percentage of intra-tumoral CLEVER-1 expressing cells from the total amount of viable intra-tumoral cells present in the stained sample, and
- beginning an administration of an anti-CLEVER-1 antibody, preferably anti-CLEVER-1 antibody bexmarilimab in a patient, when the tumor sample shows low percentage of PD-L1 expressing cells or not comprising PD-L1 expressing cells together with moderate to high percentage of CLEVER-1 expressing intra-tumoral cells.

The present invention is based on the findings that selection of patients that benefit from anti-CLEVER-1 therapy is more accurate and efficient when using the combination of PD-L1 and CLEVER-1 expression and/or the ratio of PD-L1/intra-tumoral CLEVER-1 expression measured from a tumor sample. Based on the results, intra-tumoral CLEVER-1 expression levels alone were statistically significant to identify patients that benefit from anti-CLEVER-1 therapy from those that do not, but combination of PD-L1 and CLEVER-1 expressions and/or the ratio of PD-L1/intra-tumoral CLEVER-1 expression was even better than CLEVER-1 expression alone and enables the more accurate method for pre-treatment identification of the patients. According to the present invention a tumour sample which shows no expression or low expression of PD-L1 together with moderate to high CLEVER-1 intra-tumoral expression is indicative that the cancer patient is responsive to the anti-CLEVER-1 therapy.

Based on the present invention, anti-CLEVER-1 antibody, preferably anti-CLEVER-1 antibody bexmarilimab can be efficiently used in the treatment of cancer in a patient.

### Brief Description of the drawings

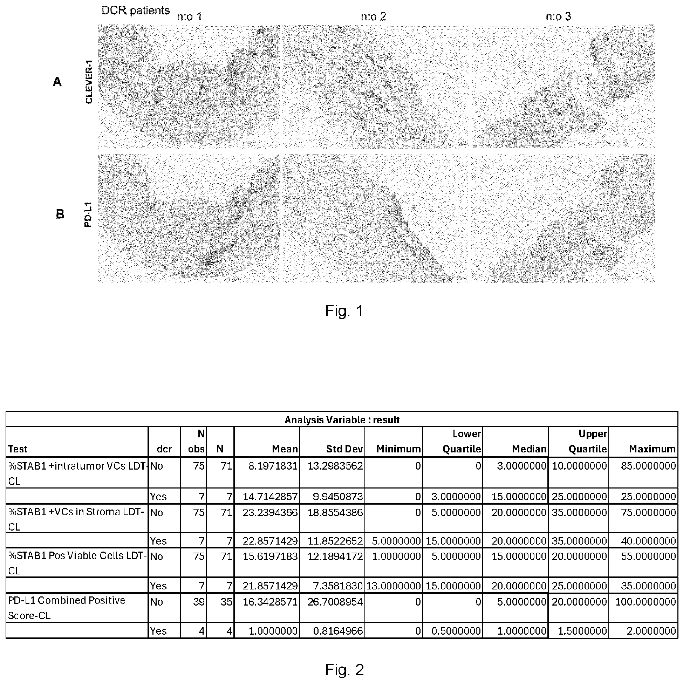

**Figures 1A** - **1B****.** Pre-treatment tumor biopsies from a first-in-human anti-CLEVER-1 trial named MATINS (clinicaltrials.gov NCT 03733990) from patients that a clinical benefit (extension of survival) from anti-CLEVER-1 treatment has observed. Figure 1A) CLEVER-1 staining, black or dark grey (brown colour in actual) identifying CLEVER-1 positive cells; Figure 1B) black or dark grey (brown colour in actual) identifying PD-L1 positive cells. Patients that see a clinical benefit from anti-CLEVER-1 treatment have a substantial amount of CLEVER-1 positive intra-tumoral cells and a very low amount of PD-L1 positive cells.

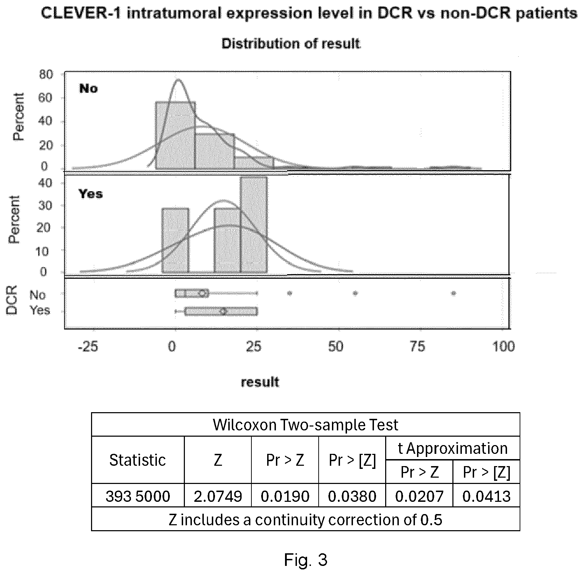

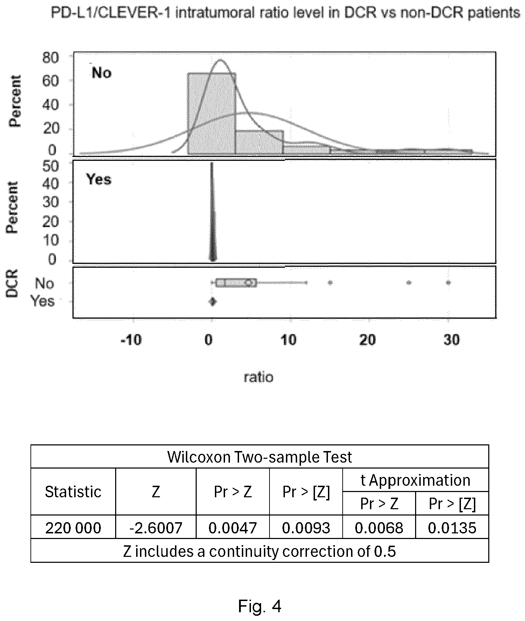

**Figures 2** - **4****.** The percentage viable cells (VCs) that are positive for CLEVER-1 and PD-L1 staining from the amount of total cells in a biopsy obtained from patient prior to anti-CLEVER-1 therapy in the MATINS Trial, and their association with clinical benefit (DCR = disease control rate). The percentage of CLEVER-1 positive intra-tumoral, but not stromal or all cells from biopsy associate with DCR (p = 0.038 in Fig. 3). PD-L1/intra-tumoral CLEVER-1 ratio is highly significant in separating non-DCR from DCR patients (p < 0.01 in Fig. 4).

### Detailed description of the invention

CLEVER-1 is a protein disclosed in the patent publication WO 03/057130, Common Lymphatic Endothelial and Vascular Endothelial Receptor-1. It is a binding protein that mediates adhesion of lymphocytes (and malignant tumor cells) to endothelium in both the systemic vasculature and in the lymphatics. CLEVER-1 (also known as STABILIN-1) is a multifunctional molecule conferring scavenging ability on a subset of anti-inflammatory macrophages [5, 6]. In these cells, it is involved in receptor-mediated endocytosis and recycling, intracellular sorting, and transcytosis of altered and normal self-components. More recently, it has been found that the progression of melanoma tumor growth and metastasis is attenuated in Stab1^{-/-} (Clever-1 knock out) mice, and in mice treated with anti-Clever-1 therapy [7]. More recently, similar results have now been achieved also in melanoma patients, as well as other cancers [2] using a novel humanized anti-CLEVER-1 antibody (FP-1305) named bexmarilimab disclosed in patent publication WO2017/182705. By blocking the interaction of CLEVER-1 and its lymphocyte substrate, it is possible to simultaneously control lymphocyte recirculation and lymphocyte migration, and related conditions such as inflammation, at the site of lymphocyte influx into, and efflux from, the tissues.

The term "an antibody or fragment(s) thereof" is used in the broadest sense to cover an antibody or fragment(s) thereof which are capable to bind CLEVER-1 molecule in an individual. Especially, it shall be understood to include chimeric, humanized or primatized antibodies, as well as antibody fragment(s) and single chain antibodies (e.g. Fab, Fv), so long they exhibit the desired biological activities. Particularly, anti-CLEVER-1 antibodies and fragment(s) thereof. According to the present invention "anti-CLEVER-1 treatment" or "anti-CLEVER-1 therapy" refers to the treatment comprising administration of at least one anti-CLEVER-1 antibody.

According to an embodiment of the invention, an anti-CLEVER-1 antibody is a therapeutic humanized anti-CLEVER-1 antibody. According to an embodiment of the present invention an anti-CLEVER-1 antibody is a humanized monoclonal anti-CLEVER-1 antibody, previously presented in the patent publication WO2017/182705.

In an embodiment of the present invention, an anti-CLEVER-1 antibody is a humanized monoclonal immunoglobulin G4 kappa antibody bexmarilimab (International Nonproprietary Name (INN)) as disclosed in WHO Drug Information, Vol. 34, No. 3 (2020), pages 699-700), or bexmarilimab variant or the antibody in a bexmarilimab biosimilar. As used herein, "bexmarilimab" means the humanized monoclonal IgG4 antibody with the structure described in WHO Drug Information, Vol. 34, No. 3 (2020).

A bexmarilimab biosimilar means a biological product which is approved by a regulatory agency in any country for marketing as a bexmarilimab biosimilar. In an embodiment, a bexmarilimab biosimilar comprises a bexmarilimab variant as the drug substance. In an embodiment, a bexmarilimab biosimilar has substantially the same amino acid sequence of heavy and light chains as bexmarilimab. As used herein, a "bexmarilimab variant" means an antibody which comprises sequences of heavy chain and light chain that are identical to those in bexmarilimab, except for having one or more conservative amino acid substitutions at positions that are located outside of the light chain CDRs and/or one or more conservative amino acid substitutions that are located outside of the heavy chain CDRs, e.g. the variant positions are located in the framework regions or the constant region. In other words, bexmarilimab and a bexmarilimab variant comprise identical CDR sequences, but differ from each other due to having a conservative amino acid substitution at other positions in their full-length light and heavy chain sequences. A bexmarilimab variant is substantially the same as bexmarilimab with respect to binding affinity to CLEVER-1.

According to an embodiment of the present invention, a cell line producing the therapeutic anti-CLEVER-1 antibody bexmarilimab (FP-1305) has been deposited on 27 May 2020 under the terms of the Budapest Treaty on the International Recognition of the Deposit of Micro-organisms for the Purposes of Patent Procedure with the DSMZ-German Collection of Microorganisms and Cell Cultures GmbH, Inhoffenstrasse 7B, D-38124 Braunschweig, Germany, and has the accession number DSM ACC3361. The present invention is not to be limited in scope by the culture deposited, since the deposited embodiment is intended as a single illustration of one aspect of the invention and any culture that is functionally equivalent is within the scope of this invention. The deposit of material herein does not constitute an admission that the written description herein contained is inadequate to enable the practice of any aspect of the invention, including the best mode thereof, nor is it to be construed as limiting the scope of the claims to the specific illustration that it represents.

In the present invention, it has been found an efficient method for selecting cancer patients that respond to anti-CLEVER-1 treatment comprising an administration of anti-CLEVER-1 antibody, preferably anti-CLEVER-1 antibody bexmarilimab.

In a method according to the present invention a tumor sample is obtained from a cancer patient prior to the beginning of anti-CLEVER-1 treatment comprising an administration of anti-CLEVER-1 antibody, preferably anti-CLEVER-1 antibody bexmarilimab in a patient, and based on the tumor sample it is evaluated the amount of the cells expressing PD-L1 (percentage of positive cells from all viable cells) and the amount of the intra-tumoral cells expressing CLEVER-1 (percentage of positive cells from all viable intratumoral cells) for making the pre-treatment identification and patient selection of cancer patients that respond to the anti-CLEVER-1 treatment.

According to an embodiment of the present invention, a tumor sample is a tumor biopsy sample. There are many different types of biopsy procedures for obtaining a tumor sample. A biopsy may be the removal of a small piece of tissue or a sample of cells.

In a method according to the present invention, both PD-L1 expression and CLEVER-1 expression are detected in the cells of the tumor sample by immunohistochemistry staining by antibodies. In a method according to the invention, the PD-L1 expressing cells is detected by staining the cells of the sample with a PD-L1 specific antibody, and the CLEVER-1 expressing cells is detected by staining the cells of the sample with a mouse monoclonal IgG2a kappa STAB-1 antibody (clone 4G9). Further, a method according to the present invention for pre-treatment identification of cancer patients comprises calculating a percentage of PD-L1 expressing cells from the total amount of viable cells present in the stained tumor sample, and calculating a percentage of intra-tumoral CLEVER-1 expressing cells from the total amount of viable intra-tumoral cells present in the stained tumor sample, wherein the stained tumor sample which shows low percentage of PD-L1 expressing cells or not comprising PD-L1 expressing cells together with moderate to high percentage of CLEVER-1 expressing intra-tumoral cells is an indication that the cancer patient is responsive to the anti-CLEVER-1 therapy. Further, a method may further comprise calculating a ratio of PD-L1 expression and intra-tumoral CLEVER-1 expression, wherein a low ratio of PD-L1 expression and CLEVER-1 intra-tumoral expression in immunohistochemistry staining of a tumor biopsy prior to the beginning of anti-CLEVER-1 therapy is an indication that the cancer patient is responsive to the anti-CLEVER-1 treatment.

More particularly, PD-L1 expression in the sample is calculated using combined positive score (CPS), which is the number of PD-L1 staining cells (tumor cells, lymphocytes and macrophages) divided by the total number of viable tumor cells in the sample, multiplied by 100. In the method according to the present invention, CLEVER-1 expression is calculated only CLEVER-1 expressing intra-tumoral cells divided by the total number of viable intra-tumoral cells in the sample, multiplied by 100. According to an embodiment of the present invention intra-tumoral cells comprises macrophages and/or tumor endothelial cells.

According to the present invention, PD-L1 expression can be evaluated or detected e.g. by using of different staining platforms and antibodies. The evaluation of PD-L1 expression can be performed any suitable method and by any suitable antibody specific for PD-L1. Several commercial diagnostic assays for determining PD-L1 expression are available and some of them are specific for certain cancer types or intended to use in combination with specific anti-PD-L1 inhibitor. In an embodiment of the method according to the invention, a PD-L1 antibody can be chosen depending on the cancer type.

In the pre-treatment screening method according to the present invention, it has been observed that for CLEVER-1 staining a highly specific anti-CLEVER-1 antibody, more particularly a mouse monoclonal IgG2a kappa STAB-1 antibody (clone 4G9) is needed for proper staining of CLEVER-1 positive cells. A monoclonal IgG2a kappa STAB-1 antibody (clone 4G9) is produced in mouse clone 4G9. A mouse monoclonal IgG2a kappa STAB-1 antibody (clone 4G9) is raised against amino acids 1804-1902 of STABILIN-1 (CLEVER-1) of human origin (SEQ ID NO: 1). In an exemplary embodiment, anti-CLEVER-1 antibody used in the method according to the present invention is STAB1 monoclonal antibody (M05), clone 4G9 from Abnova (Taiwan) Corporation (product details: http://www.abnova.com/products/products_detail.asp?catalog_id=H0002316 6-M05). The amount of CLEVER-1 expressing cells from sample may be calculated by manually by pathologist using microscopy, but for achieving more accurate results, samples are stained and machine read by automated systems. Hence, the staining need to be done by antibody which is suitable for machine reading and do not cause background color which may disturb machine reading of the stained samples. In the present invention, it has been found that a mouse monoclonal IgG2a kappa STAB-1 antibody (clone 4G9) provides accurate staining without interfering background color, and therefore the proper staining for pre-treatment evaluation is achieved by a mouse monoclonal IgG2a kappa STAB-1 antibody (clone 4G9) and the calculating of the CLEVER-1 expressing cells can be done by machine reading accurately.

Commonly, PD-L1 expression on cells from a tumor biopsy are used to predict patient eligibility for treatment with PD-1 inhibitors. A level of PD-L1 expression may vary based on the type of cells in which PD-L1 is assessed (tumor versus immune cells), or the source and timing for sample collection. The PD-L1 expression (CPS) in ≥ 50 % of the viable cells (tumor cells, lymphocytes and macrophages) present in the sample is typically considered as high expression for PD-L1 and it predicts responsiveness to PD-1/PD-L1 inhibitors. The PD-L1 expression (CPS) in less than 1 % of the viable cells present in the sample is considered as no expression, and PD-L1 expression (CPS) in the range of 1-49 % of the viable cells present in the sample is also considered as PD-L1 positive, but the responsiveness might be dependent on the therapy.

The first human data using a humanized anti-CLEVER-1 antibody (bexmarilimab) from trial MATINS indicates that patients that see a clinical benefit (tumor shrinkage or stable disease) from anti-CLEVER-1 therapy comprising an administration of anti-CLEVER-1 antibody bexmarilimab have PD-L1 expression of 0-2% (median 1%), calculated from the total amount of viable cells present in the stained sample, while patient that do not respond to anti-CLEVER-1 therapy may have PD-L-1 expression from 0-100% (median 5%), calculated from the total amount of viable cells present in the stained sample (Figure 2). According to an embodiment of the present invention, low percentage of PD-L1 expressing cells is 0-2%, calculated from the total amount of viable cells present in the stained sample, which together with moderate to high CLEVER-1 expression level is an indication that the cancer patient is responsive to the anti-CLEVER-1 treatment.

According to an embodiment of the present invention, an amount of CLEVER-1 expressing intra-tumoral cells is at least 1 %, calculate from the total amount of viable intra-tumoral cells present in the stained sample.

The administration of anti-CLEVER-1 antibody bexmarilimab is most valuable for patients having diagnosed with a tumour which shows no expression or low expression of PD-L1 in addition to a substantial amount of CLEVER-1 positive intra-tumoral cells.

Hence, according to an embodiment of the present invention PD-L1 expression and intra-tumoral CLEVER-1 expression can be used as the predictive biomarkers prior to the treatment for determining patient's responsiveness to anti-CLEVER-1 therapy. The patients which typically responded well to anti-PD-1 therapy had PD-L1-positive and T-lymphocyte-rich tumor specimen. According to an embodiment of the present invention, the expression of PD-L1 may be tested by staining tumor cells with anti-PD-L1 antibody. PD-L1 protein expression is typically determined by calculating the percentage of viable tumor cells showing partial or complete membrane staining at any intensity from total amount of the of viable cells present in the sample, while for CLEVER-1 staining only the amount of positive cells that are intra-tumoral are meaningful. In the method according to the present invention, the percentage of PD-L1 expression level is calculated from the total amount of viable cells present in the stained sample and the percentage of CLEVER-1 intra-tumoral expression level is calculated from the total amount of intra-tumoral cells.

According to an embodiment of the present invention, the method comprises calculating a ratio of PD-L1 expressing cells and intra-tumoral CLEVER-1 expressing cells in the stained sample, wherein low PD-L1/intra-tumoral CLEVER-1 ratio is an indication that the cancer patient is responsive to the anti-CLEVER-1 treatment. PD-L1/intra-tumoral CLEVER-1 ratio is calculated from the percentages of PD-L1 expressing cells and CLEVER-1 intra-tumoral expressing cells. According to an embodiment of the present invention, the stained tumor sample, which shows 0 - 2 % of PD-L1 expressing cells, calculated from the total amount of viable cells present in the stained sample, together with at least 1 % of CLEVER-1 expressing intra-tumoral cells, calculated from the total amount of viable intra-tumoral cells present in the stained sample, is an indication that the cancer patient is responsive to the anti-CLEVER-1 therapy. Hence, according to an embodiment of the present invention a ratio of PD-L1 expression and intra-tumoral CLEVER-1 expressions is ≤ 2, preferably < 2, calculated based on the percentage values, wherein it is an indication that the cancer patient is responsive to the anti-CLEVER-1 treatment.

In the method according to the present invention, a decision to start an anti-CLEVER-1 therapy is made based on the pre-treatment diagnosis. A tumour which shows no expression or low expression of PD-L1 together moderate to high CLEVER-1 expression is indicative that the patient is responsive to the anti-CLEVER-1 therapy. Anti-CLEVER-1 therapy or treatment refers to a treatment comprising an administration of anti-CLEVER-1 antibody, preferably anti-CLEVER-1 antibody bexmarilimab. In a typical method for treating a cancer patient, anti-CLEVER-1 antibody is administered therapeutically effective amount.

The term "treatment" or "treating" shall be understood to include complete curing of a disease or disorder, as well as amelioration or alleviation of said disease or disorder. The term "therapeutically effective amount" is meant to include any amount of an agent that is sufficient to bring about a desired therapeutic result. "Administering" refers to the physical introduction of a composition comprising said therapeutic agents to an individual, using any of the various methods and delivery systems known to those skilled in the art. The agents to be may be administered by any means that achieve their intended purpose. For example, administration may be intravenous, intramuscular, intraperitoneal, intra-tumoral, subcutaneous or other parenteral routes of administration, for example by injection. In addition to the pharmacologically active compounds, the pharmaceutical preparations of said agents preferably contain suitable pharmaceutically acceptable carriers comprising excipients and auxiliaries that facilitate processing of the active agents into preparations that can be used pharmaceutically. The dose chosen should be sufficient to reduce malignant tumor growth and/or inhibit metastasis formation.

Treating cancer by reducing malignant tumor growth and/or by inhibiting metastasis formation is applicable to all forms of cancers. Thus, any benign or malignant tumor or metastasis of malignant tumor can be treated.

### EXPERIMENTAL PART

### First-in-human clinical study of an anti-CLEVER-1 antibody

CLEVER-1 inhibiting agent, an anti-CLEVER-1 antibody FP-1305, is currently being tested for safety and preliminary efficacy in a Phase I/II study in patient with advanced solid tumors (clinicaltrials.gov NCT03733990: A Study to Evaluate Safety, Tolerability and Preliminary Efficacy of FP-1305 in Cancer Patients (MATINS)).

The anti-CLEVER-1 antibody FP-1305 is a humanized monoclonal CLEVER-1 antibody, previously presented in the patent publication WO2017/182705. More precisely, FP-1305 (DSM ACC3361) is a humanized monoclonal immunoglobulin G4κ antibody bexmarilimab (International Nonproprietary Name (INN)) as disclosed in WHO Drug Information, Vol. 34, No. 3 (2020), pages 699-700) produced in CHO cells.

In the present study, a pre-treatment tumour biopsy is obtained prior to starting anti-CLEVER-1 antibody FP-1305 treatment and fixated in formalin (FFPE) for later immunohistochemistry (IHC) staining and analysis by an independent centralized pathologist. Tumour progression or regression is evaluated repeating a CT scan that is compared to an existing scan taken before initiating anti-CLEVER-1 treatment. Progressive disease (PD) means the cancer is growing. No significant change in tumour size, which is a positive effect in aggressive otherwise non-treatable cancers, such as in the MATINS trial, is labelled as stable disease (SD), and considered good response. Tumour shrinkage is referred to as partial response (PR) according to the RECIST criteria used to evaluate treatment response. DCR (DCR = disease control rate) patients are patients who have achieved complete response, partial response and stable disease to a therapeutic intervention of anti-CLEVER-1 antibody FP-1305.

### IHC staining of tumor biopsies prior to anti-CLEVER-1 therapy

FFPE (formalin-fixed paraffin-embedded) tumor samples obtained from MATINS Trial patients of 4-5 µm sections were stained with Ventana Benchmark Ultra (Roche Diagnostics, Basel, Switzerland). For CLEVER-1 stainings, UltraView Universal DAB Detection Kit (Roche Diagnostics) combined with CLEVER-1 primary antibody (monoclonal IgG2a kappa STAB-1 antibody (clone 4G9), Abnova (Taiwan) Corporation) at 1:100 dilution was used. PD-L1 staining was performed using 22C3 pharmDx assay (Agilent Technologies, Santa Clara, CA, USA) according to manufacturer's instructions. CLEVER-1 and PD-L1 stainings from DCR patients are shown in Figure 1. Interpretation and scoring were performed by board-certified pathologists using bright field microscopy. Percentage of CLEVER-1 positive viable cells (the number of all CLEVER-1 positive cells divided by the total number of cells, multiplied by 100) were scored irrespectively of location, and also percentage of CLEVER-1 positive viable cells were scored intratumorally and in stroma. PD-L1 was scored as combined positive score (CPS) by calculation the number of PD-L1 staining cells (tumor cells, lymphocytes, macrophages) divided by the total number of viable tumor cells, multiplied by 100. Results are shown in Figure 2.

Figure 2 shows the percentage viable cells (VCs) that are positive for CLEVER-1 and PD-L1 staining from the amount of total cells in a biopsy obtained from patient prior to anti-CLEVER-1 therapy in the MATINS Trial, and their association with clinical benefit (DCR patients). Also, a percentage of CLEVER-1 expressing cells in stromal and intra-tumoral viable cells are shown. It has been observed that DCR patients had higher intra-tumoral CLEVER-1 staining and low PD-L1 staining.

Based on the results, intra-tumoral CLEVER-1 expression levels alone were statistically significant to identify patients that benefit from anti-CLEVER-1 therapy from those that do not (p = 0.038) (Fig. 3), but the ratio of PD-L1/intra-tumoral CLEVER-1 expression was even better than CLEVER-1 expression alone (p < 0.01) (Fig 4). Hence, according to the present invention tumors showing no PD-L1 expression or low PD-L1 expression together with medium to high CLEVER-1 expression levels of tumor infiltrating cells in the biopsy sample are an indication to responsiveness to the therapy. The percentage of expression levels is calculated from the total amount of viable cells present in the stained sample for PD-L1 and for intra-tumoral cells only for CLEVER-1. PD-L1/intra-tumoral CLEVER-1 ratio is highly significant in separating non-DCR from DCR patients (p < 0.01 in Fig. 4), and hence predicting patients that benefit from anti-CLEVER-1 therapy.

### Cited references:

[1] Chen DS, Mellman I. Elements of cancer immunity and the cancer-immune set point. Nature 2017; 541(7637): 321-30.
[2] Virtakoivu et al. Systemic Blockade of Clever-1 Elicits Lymphocyte Activation Alongside Checkpoint Molecule Downregulation in Patients with Solid Tumors: Results from a Phase I/II Clinical Trial. Clin Cancer Res 2021; 27:4205-20.
[3] Guerriero JL, Sotayo A, Ponichtera HE, Castrillon JA, Pourzia AL, Schad S, et al. Class IIa HDAC inhibition reduces breast tumours and metastases through anti-tumour macrophages. Nature 2017; 543(7645):428-32.
[4] Qian BZ, Pollard JW. Macrophage diversity enhances tumor progression and metastasis. Cell 2010; 141(1):39-51.
[5] Kzhyshkowska J, Gratchev A, Goerdt S. Stabilin-1, a homeostatic scavenger receptor with multiple functions. J Cell Mol Med 2006;10(3):635-49.
[6] Kzhyshkowska J, Workman G, Cardó-Vila M, Arap W, Pasqualini R, Gratchev A, et al. Novel function of alternatively activated macrophages: stabilin-1-mediated clearance of SPARC. J Immunol 2006;176(10): 5825-32.
[7] Karikoski M, Marttila-Ichihara F, Elima K, Rantakari P, Hollmen M, Kelkka T, et al. Clever-1/Stabilin-1 Controls Cancer Growth and Metastasis. Clinical Cancer Research 2014;20(24):6452-64.
[8] WO 2010/122217 A1

## Claims

1. A method for pre-treatment identification of cancer patient that respond to anti-CLEVER-1 therapy comprising an administration of an agent capable of binding to Common Lymphatic Endothelial and Vascular Endothelial Receptor-1 (CLEVER-1) in a patient, **characterized in that** the method comprises
- providing a tumor sample obtained from a cancer patient,
- detecting the presence of PD-L1 expressing cells and CLEVER-1 expressing cells in the tumor sample by immunohistochemistry staining by a PD-L1 specific antibody and a mouse monoclonal IgG2a kappa STAB-1 antibody (clone 4G9), and
- calculating a percentage of PD-L1 expressing cells from the total amount of viable cells present in the stained sample, and calculating a percentage of intra-tumoral CLEVER-1 expressing cells from the total amount of viable intra-tumoral cells present in the stained sample,
wherein the tumor sample, which shows low percentage of PD-L1 expressing cells or not comprising PD-L1 expressing cells together with substantial percentage of CLEVER-1 expressing intra-tumoral cells is an indication that the cancer patient is responsive to the anti-CLEVER-1 therapy.

2. The method according to claim 1, **characterized in that** the method comprises calculating a ratio of PD-L1 expressing cells and intra-tumoral CLEVER-1 expressing cells in the stained sample, wherein low PD-L1/intra-tumoral CLEVER-1 ratio is an indication that the cancer patient is responsive to the anti-CLEVER-1 therapy.

3. The method according to claim 1 or 2, **characterized in that** the agent capable of binding to CLEVER-1 comprises anti-CLEVER-1 antibody, preferably anti-CLEVER-1 antibody bexmarilimab.

4. The method according to any one of the preceding claims, **characterized in that** the tumor sample is a tumor biopsy sample.

5. The method according to any one of the preceding claims, **characterized in that** intra-tumoral cells comprise macrophages and/or tumor endothelial cells.

6. The method according to any one of the preceding claims, **characterized in that** the low percentage of PD-L1 expressing cells is 0-2%, calculated from the total amount of viable cells present in the stained sample.

7. The method according to any one of the preceding claims, **characterized in that** the substantial percentage of CLEVER-1 expressing cells is at least 1 %, calculate from the total amount of viable intra-tumoral cells present in the stained sample.

8. An anti-CLEVER-1 antibody for use in a treatment of cancer in an individual, which is identified by the method according to any of the preceding claims 1 - 7.

9. The anti-CLEVER-1 antibody for use in a treatment of cancer in an individual according to claim 8, **characterized in that** the anti-CLEVER-1 antibody comprises anti-CLEVER-1 antibody bexmarilimab.

## Patentansprüche

1. Verfahren zur Identifizierung von Krebspatienten vor der Behandlung, die auf eine Anti-CLEVER-1-Therapie ansprechen, umfassend die Verabreichung eines Mittels, das in der Lage ist, an den Common Lymphatic Endothelial and Vascular Endothelial Receptor-1 (CLEVER-1) zu binden, an einen Patienten, **dadurch gekennzeichnet, dass** das Verfahren Folgendes umfasst
- Bereitstellen einer Tumorprobe, die von einem Krebspatienten erhalten wurde,
- Nachweis des Vorhandenseins von PD-L1-exprimierenden Zellen und CLEVER-1-exprimierenden Zellen in der Tumorprobe durch immunhistochemische Färbung mit einem PD-L1-spezifischen Antikörper und einem monoklonalen IgG2a kappa STAB-1-Antikörper der Maus (Klon 4G9), und
- Berechnen eines Prozentsatzes von PD-L1-exprimierenden Zellen aus der Gesamtmenge der lebensfähigen Zellen, die in der gefärbten Probe vorhanden sind, und Berechnen eines Prozentsatzes von intra-tumoralen CLEVER-1-exprimierenden Zellen aus der Gesamtmenge der lebensfähigen intra-tumoralen Zellen, die in der gefärbten Probe vorhanden sind,
wobei die Tumorprobe, die einen niedrigen Prozentsatz von PD-L1-exprimierenden Zellen aufweist oder keine PD-L1-exprimierenden Zellen zusammen mit einem wesentlichen Prozentsatz von CLEVER-1-exprimierenden intra-tumoralen Zellen umfasst, ein Hinweis darauf ist, dass der Krebspatient auf die Anti-CLEVER-1-Therapie anspricht.

2. Verfahren nach Anspruch 1, **dadurch gekennzeichnet, dass** das Verfahren das Berechnen eines Verhältnisses von PD-L1-exprimierenden Zellen und intra-tumoralen CLEVER-1-exprimierenden Zellen in der gefärbten Probe umfasst, wobei ein niedriges PD-L1/intra-tumorales CLEVER-1-Verhältnis ein Hinweis darauf ist, dass der Krebspatient auf die Anti-CLEVER-1-Therapie anspricht.

3. Verfahren nach Anspruch 1 oder 2, **dadurch gekennzeichnet, dass** das Mittel, das an CLEVER-1 binden kann, einen Anti-CLEVER-1-Antikörper, vorzugsweise den Anti-CLEVER-1-Antikörper Bexmarilimab, umfasst.

4. Verfahren nach einem der vorhergehenden Ansprüche, **dadurch gekennzeichnet, dass** die Tumorprobe eine Tumorbiopsieprobe ist.

5. Verfahren nach einem der vorhergehenden Ansprüche, **dadurch gekennzeichnet, dass** intra-tumorale Zellen Makrophagen und/oder Tumorendothelzellen umfassen.

6. Verfahren nach einem der vorhergehenden Ansprüche, **dadurch gekennzeichnet, dass** der niedrige Prozentsatz an PD-L1-exprimierenden Zellen 0-2 % beträgt, berechnet aus der Gesamtmenge der lebensfähigen Zellen, die in der gefärbten Probe vorhanden sind.

7. Verfahren nach einem der vorhergehenden Ansprüche, **dadurch gekennzeichnet, dass** der wesentliche Prozentsatz der CLEVER-1-exprimierenden Zellen mindestens 1 % beträgt, berechnet aus der Gesamtmenge der lebensfähigen intra-tumoralen Zellen, die in der gefärbten Probe vorhanden sind.

8. Anti-CLEVER-1-Antikörper zur Verwendung bei einer Behandlung von Krebs bei einem Individuum, das durch das Verfahren nach einem der vorhergehenden Ansprüche 1 - 7 identifiziert wird.

9. Anti-CLEVER-1-Antikörper zur Verwendung bei einer Behandlung von Krebs bei einem Individuum gemäß Anspruch 8, **dadurch gekennzeichnet, dass** der Anti-CLEVER-1-Antikörper Anti-CLEVER-1-Antikörper Bexmarilimab umfasst.

## Revendications

1. Procédé d'identification de pré-traitement de patients atteints d'un cancer qui répondent à une thérapie anti-CLEVER-1 comprenant l'administration d'un agent capable de se lier au récepteur endothélial lymphatique et endothélial vasculaire commun de type 1 (CLEVER-1) chez un patient, **caractérisé en ce que** le procédé comprend
- fournir un échantillon de tumeur obtenu chez un patient atteint de cancer,
- détecter la présence de cellules exprimant PD-L1 et de cellules exprimant CLEVER-1 dans l'échantillon de tumeur par coloration immunohistochimique avec un anticorps spécifique du PD-L1 et un anticorps monoclonal de souris IgG2a kappa STAB-1 (clone 4G9), et
- calculer un pourcentage de cellules exprimant PD-L1 à partir de la quantité totale de cellules viables présentes dans l'échantillon coloré, et calculer un pourcentage de cellules intra-tumorales exprimant CLEVER-1 à partir de la quantité totale de cellules intra-tumorales viables présentes dans l'échantillon coloré,
dans lequel l'échantillon de tumeur, qui présente un faible pourcentage de cellules exprimant PD-L1 ou ne comprenant pas de cellules exprimant PD-L1 avec un pourcentage substantiel de cellules intra-tumorales exprimant CLEVER-1, est une indication que le patient atteint d'un cancer est sensible à la thérapie anti-CLEVER-1.

2. Procédé selon la revendication 1, **caractérisé en ce que** le procédé comprend le calcul d'un rapport de cellules exprimant PD-L1 et de cellules intra-tumorales exprimant CLEVER-1 dans l'échantillon coloré, dans lequel un faible rapport PD-L1/CLEVER-1 intra-tumoral est une indication que le patient atteint d'un cancer est sensible à la thérapie anti-CLEVER-1.

3. Procédé selon la revendication 1 ou 2, **caractérisé en ce que** l'agent capable de se lier à CLEVER-1 comprend un anticorps anti-CLEVER-1, de préférence un anticorps anti-CLEVER-1 bexmarilimab.

4. Procédé selon l'une quelconque des revendications précédentes, **caractérisé en ce que** l'échantillon de tumeur est un échantillon de biopsie tumorale.

5. Procédé selon l'une quelconque des revendications précédentes, **caractérisé en ce que** les cellules intra-tumorales comprennent des macrophages et/ou des cellules endothéliales tumorales.

6. Procédé selon l'une quelconque des revendications précédentes, **caractérisé en ce que** le faible pourcentage de cellules exprimant PD-L1 est de 0 à 2 %, calculé à partir de la quantité totale de cellules viables présentes dans l'échantillon coloré.

7. Procédé selon l'une quelconque des revendications précédentes, **caractérisé en ce que** le pourcentage substantiel de cellules exprimant CLEVER-1 est d'au moins 1 %, calculé à partir de la quantité totale de cellules intra-tumorales viables présentes dans l'échantillon coloré.

8. Anticorps anti-CLEVER-1 pour une utilisation dans un traitement du cancer chez un individu, qui est identifié par le procédé selon l'une quelconque des revendications précédentes 1 à 7.

9. Anticorps anti-CLEVER-1 pour une utilisation dans un traitement du cancer chez un individu selon la revendication 8, **caractérisé en ce que** l'anticorps anti-CLEVER-1 comprend l'anticorps anti-CLEVER-1 bexmarilimab.
